(19)

**Europäisches Patentamt**
**European Patent Office**
**Office européen des brevets**

(11) **EP 4 483 785 B1**

(12) **EUROPEAN PATENT SPECIFICATION**

(45) Date of publication and mention
of the grant of the patent:
**21.01.2026 Bulletin 2026/04**

(51) International Patent Classification (IPC):
**A61B 5/00** (2006.01)    **A61B 5/026** (2006.01)
**A61B 8/06** (2006.01)

(21) Application number: **23182135.6**

(22) Date of filing: **28.06.2023**

(52) Cooperative Patent Classification (CPC):
**A61B 5/0053; A61B 5/026; A61B 5/4064;**
**A61B 5/746; A61B 8/06;** A61B 2505/05

(54) **METHOD AND SYSTEM FOR ESTIMATING STATUS OF HUMAN BRAIN CEREBRAL BLOOD FLOW AUTOREGULATION FOR PERSONALIZED BRAIN PERFUSION MANAGEMENT**

VERFAHREN UND SYSTEM ZUR SCHÄTZUNG DES ZUSTANDS DER AUTOREGULATION DES ZEREBRALEN BLUTFLUSSES IM MENSCHLICHEN GEHIRN FÜR PERSONALISIERTES GEHIRNPERFUSIONSMANAGEMENT

PROCÉDÉ ET SYSTÈME D'ESTIMATION DE L'ÉTAT D'AUTORÉGULATION DU DÉBIT SANGUIN CÉRÉBRAL HUMAIN POUR LA GESTION PERSONNALISÉE DE PERFUSION CÉRÉBRALE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(43) Date of publication of application:
**01.01.2025 Bulletin 2025/01**

(73) Proprietor: **Kaunas University of Technology**
**44029 Kaunas (LT)**

(72) Inventors:
• **Petkus, Vytautas**
**LT-48300 Kaunas (LT)**
• **Chaleckas, Edvinas**
**LT-50374 Kaunas (LT)**
• **Ragauskas, Arminas**
**LT-51362 Kaunas (LT)**

(74) Representative: **Klimaitiene, Otilija**
**AAA Law**
**A. Gostauto 40B**
**03163 Vilnius (LT)**

(56) References cited:
**CN-A- 106 264 513     US-A1- 2015 366 514**

• **AASLID R ET AL: "Cerebral autoregulation dynamics in humans.", vol. 20, no. 1, 1 January 1989 (1989-01-01), pages 45 - 52, XP093095465, Retrieved from the Internet <URL:https://www.ahajournals.org/doi/pdf/10.1161/01.STR.20.1.45>**
• **RONNEY B PANERAI ET AL: "TOPICAL REVIEW; Assessment of cerebral pressure autoregulation in humans - a review of measurement methods", PHYSIOLOGICAL MEASUREMENT, INSTITUTE OF PHYSICS PUBLISHING, BRISTOL, GB, vol. 19, no. 3, 1 August 1998 (1998-08-01), pages 305 - 338, XP020073857, ISSN: 0967-3334, DOI: 10.1088/0967-3334/19/3/001**

EP 4 483 785 B1

**Description**

FIELD OF INVENTION

The present invention is directed to a method and system of human brain cerebral blood flow autoregulation status (CA-status) identification for personalized brain perfusion management.

BACKGROUND OF THE INVENTION

Cerebral blood flow (CBF) autoregulation means the maintenance of close-to-constant cerebral blood flow across a range of patient-specific cerebral perfusion pressures (CPP). Autoregulation is a homeostatic function that protects the brain from ischemic and hyperemic injuries caused by too low or too high cerebral perfusion pressure in a specific individual patient. Real-time monitoring of the status of quasistatic ("static" in medical literature) cerebrovascular blood flow autoregulation (CA) is used for continuous long-term patient monitoring in an intensive care unit (ICU) for personalized management of patients with traumatic brain injury (TBI), patients after subarachnoid hemorrhage (SAH) or intracerebral hemorrhage (ICH). Real-time Continuous monitoring of CA-status is performed to accumulate CPP, ABP, and CA index data over a 2-8 hours time interval and to identify optimal values of cerebral perfusion pressure (CPP) or optimal arterial blood pressure (ABP) at which CA indexes show the best achievable intact autoregulation condition. Personalized CPP (or ABPopt) management is based on controlling the CPP (or ABP) values and maintaining them at patient-specific optimal values (CPPopt or ABPopt), thus avoiding cerebrovascular autoregulation (CA) impairments and the consequent damage or deaths of brain neurons.

Such CPPopt (or ABPopt) -based personalized management of patients' cerebral perfusion aims to minimize time intervals with impaired CA and minimizes the duration of such events that may cause brain injuries. During the development of the present invention, it has been discovered that a single longest CA impairment event with a duration exceeding 60 minutes is associated with fatal outcomes of TBI or SAH patients [1]. The single longest CA impairment exceeding 5 min duration is associated with brain neuron damage for patients undergoing cardiac surgery with cardiopulmonary bypass [2].

Existing technologies of personalized brain perfusion management are based on CPPopt or ABPopt measurements and estimations. They require collecting of the monitoring data for some longer periods which are mostly too long for efficient and timely medical practices.

Reliable identification of a patient's CA status requires the presence of slow waves of ABP(t) and intracranial pressure ICP(t), and the ABP changes occurring within the lower and upper limits of CA function [3]. Slow ICP waves have an intermittent nature with random variations in amplitude and oscillation frequency. An invasive CA index - Pressure Reactivity Index (PRx) - is calculated as a moving Pearson correlation coefficient between the ICP(t) and ABP(t) slow waves within a 5 min. or longer moving-average window and an additional accumulation of multimodal brain monitoring data [4]. Non-invasive CA index - mean-flow autoregulation index (Mx) - is calculated as a moving Pearson correlation coefficient between cerebral blood flow velocity BV(t) and ABP(t) slow waves within a 5 min. or longer moving-average-window [5]. Negative index PRx(t) and Mx(t) values represent the intact CA status, while positive values of PRx(t) and Mx(t) indicate the impaired CA-status. Plotting PRx, Mx against CPP or ABP will generate a "U-shaped" curve with a minimum point indicating the optimal value of CPPopt or ABPopt as a target estimate for the patients' personalized treatment.

Typically, identification of CPP and ABP optimal values (CPPopt, ABPopt) requires 4 or more hours of continuous monitoring of high-resolution multimodal data of ICP(t) and ABP(t), and monitoring the BV(t). The estimated PRx and Mx values are close to zero if the amplitude of the ICP(t) and ABP(t) slow waves is too low. Diagnostic information is lost in such cases. The "U-shaped" curve, when plotting PRx against CPP, can be obtained only when the CPP varies in a wide enough range (for example, from the lower to the upper autoregulation limits) and the PRx values are identified without unacceptable uncertainty in the presence of slow ABP(t) and ICP(t) waves of appropriate amplitude. Otherwise, the "U-shaped" curve cannot be obtained, or only a part of this curve can be identified. The quality of the "U-shaped" curve is also affected if the monitoring data used for CPPopt identification includes artifacts. All these factors can significantly affect the quality of the informative data required to identify the CPPopt value [3].

Neither known CPPopt nor ABPopt identification technologies, nor CA monitoring technologies can give instant real-time feedback and CA-status estimate, needed by medical staff to make a diagnosis. Such technologies require moving-time-averaging of the recorded data, to achieve a needed signal-to-noise ratio, thereby enabling a CA index calculation. To identify a starting point of a single longest CA impairment in real-time, a high-temporal-resolution monitoring technology is required. A time resolution of less than one minute is required for enabling brain neuroprotection, in cases of substantial CA impairments. Slow ABP and intracranial pressure (ICP) or intracranial blood volume (IBV), or blood oxygenation waves' monitoring and calculated correlation or phase shift monitoring technologies cannot be used for real-time sub-minute temporal resolution CA status monitoring.

Continuous sub-minute identification of CA-status indexes with the real-time cerebral perfusion regulation feedback was introduced in the US patent application US2023109678 A1 **[8]**. Such implementation is possible for CA status monitoring during cardiac surgery when a cardiopulmonary bypass machine for blood supply is used. The invention provides a novel rectangular mode of blood flow generated by the heart-lung machine, the ability to identify transient functions of the cerebral autoregulation system periodically with a sub-minute period, and the ability to identify a CA impairment event with a sub-minute temporal resolution The invention includes the feedback from a CA status monitor to the cardiac surgery theater by an immediate alarm after identification of CA impairment event. The alarm gives a warning triggering active steps by the caregiver or surgeon to take steps for neuroprotection of the patient's brain from postoperative cognitive dysfunction (POCD). In the preferred embodiment triggered alarm gives at least four minutes for cardiac surgeons and/or anesthetists to reestablish the patient's CA to an intact state by precise management of the arterial blood pressure of an individual patient.

Such sub-minute CA status identification and brain protection are possible only during cardiac surgeries with cardiopulmonary bypass machines when direct real-time regulation for blood flow is possible.

However, there are still no known CA-status estimation methods, the use of which would enable continuous reliable, and optimal brain perfusion regulation without using an external cardiopulmonary bypass machine.

SUMMARY OF THE INVENTION

**Technical problem.** The present invention aims to eliminate the need of collecting/accumulating the ABP slow-wave data during long periods of a few hours, for obtaining a patient's CA-status estimate which was sufficient to enable medical staff decisions regarding CPPopt-guided (or ABPopt-guided) personalized patients' treatment.

**Solution.** The present invention discloses a new technological solution - ***method and system*** - for estimating a snapshot-type CA-status-index within a 1-3 minute interval of temporal resolution. The solution uses 3-4 such CA-status-snapshots, to obtain within 10 minutes the CA-status estimate which is sufficient to enable diagnosis and decisions by medical/caregiving staff for patient's personalized treatment. The current invention is defined by the claims.

***The method*** estimates the patient's CA-status from a time delay $\Delta T$ measured between an induced arterial blood pressure ABP(t) change and the corresponding transient response of the blood velocity BV(t) in the middle cerebral artery (MCA). Those are measured during an externally initiated drop of the patient's ABP. The brain perfusion management further can be decided by medical staff, on the estimated patient's CA-status. Therefore, the CPPopt-guided (or ABPopt-guided) personalized brain perfusion management can be performed only by inducing ABP changes by external means, such as inflatable pants, and estimating the CA-status from these ABP changes, without calculating PRx or Mx indexes.

***The system*** comprises inflatable pants, to induce the patient's ABP changes by generating external pressure changes, as periodic inflation-deflation cycles, 1-3 minutes each. CA-status can be estimated by a series of 3-4 such inflations-deflations of the pants. Specific features of one embodiment of the invention include a control sub-system comprising inflatable pants, an air pump, and an external pressure control system that generates periodic pants inflation and deflation cycles, by inflating the pants with the controlled external pressure Pe. The period of the pants inflation cycle is preferably 1-3 minutes. The period of the pants deflation cycle is preferably 5-15 seconds. Cyclic inflations-deflations are applied to generate the arterial blood pressure (ABP) waves and ABP drop steps. A series of inflation-deflation cycles with different controlled external pressure peak-levels Pe1, Pe2, ... are applied to achieve mean arterial blood pressure (mABP) increments to corresponding mABP peak-levels mABP1, mABP2, ... which are above the baseline value mABP0. The higher the applied external pressure Pe for the pants inflation, the higher mABP changes (increments) are achieved. The applied external pressure Pe values are controlled within safe limits which are enough to generate a slow ABP increase wave during the inflation cycle and a fast ABP drop per ~0-20 mmHg during the pants deflation cycle.

***The system*** further comprises or is connected to any one of the CA monitor devices:

-    a non-invasive CA monitor (based on Transcranial Doppler (TCD), Ultrasonic Time of Flight (UToF)), or
-    an invasive CA monitor (based on invasive ICP measurement and pressure reactivity index PRx calculation).

Any one of those CA monitor devices continuously records the relevant transient responses (dynamic autoregulation responses) of the human CA-function. The patient's CA-status is estimated from the transient response delay time $\Delta T$ of the individual's CA-function.

According to one embodiment of the invention, the transient-response-time of the CA function is estimated non-invasively, as the delay time $\Delta T$ between cerebral blood flow velocity BV(t) change measured in the middle cerebral artery (MCA), and the relevant ABP(t) change induced by a drop of the external pressure Pe.

According to another embodiment, the transient-response-time of the CA-function is estimated as the delay time $\Delta T$ between the intracranial pressure ICP(t) change measured with invasive ventricular or parenchymal sensors, and the relevant ABP(t) change induced by a drop of the external pressure Pe.

In the present invention, it was observed that for healthy volunteers (individuals) having intact CA, the delay $\Delta T$

between the induced ABP change and the BV in MCA transient-response, during the pants deflation cycle, normally was within the 2 to 5 seconds range. For SAH and ICH patients with unfavorable outcomes, the delay between the induced ABP change and BF in MCA (or ICP) transient response, during the pants deflation cycle was observed to be less than 1.6 seconds.

*The system* generates control signals to the air pump to inflate pants, thereby inducing the ABP drops during the deflation cycles, while the preceding inflation cycles increase the ABP slowly to different ABP values (peak levels). At least, 3-4 inflation-deflation cycles with different inflation pressure Pe peak levels are necessary, to induce different amplitude drops of the mean arterial blood pressure (mABP), and then to measure corresponding time delays $\Delta T$ between the BV(t) and ABP(t) signals during the deflation cycles.

In general, the pants inflation can be performed up to two or more different Pe peak levels within safe limits. These safe limits for the maximum Pe are considered up to Pe=220 mmHg which is available in most commercially available lymphatic drainage therapy devices (pressotherapy) devices (for example, CarePump Expert8, UNIX Lympha Pro 1) used in ICU. Such pressotherapy massages are typical procedures in ICU patients used to minimize the risk of bedsores during long-term bedside treatment. The aforementioned different two or more different Pe pressure peak values should be selected such that their induced ABP changes were achieved up to 5-10 mmHg above the ABP baseline.

*The method* further uses 3-4 or more (*N*) CA-status snapshots, obtained with different *Pe* levels (at least two: *Pe1, Pe2),* to calculate a correlation coefficient *r* and a linear regression *a* between the arrays of the measured time delays $[\Delta T_1, \Delta T_2 ... \Delta T_N]$ and the measured mean-ABPs [mABP1, *mABP2...* mABPN]. The calculated values of the correlation coefficient *r* and slope of linear regression *a,* by their obtained negative or positive sign, indicate how the patient's ABP may be managed, for achieving intact CA-status and optimal brain perfusion. The positive sign indicates for that the ABP shall be raised, and the negative sign indicates for that the ABP shall be reduced. These indications for the ABP raise or reduction should be reviewed by medical/caregiving staff (nurses, anesthetists, and surgeons), which would take appropriate measures for personalized ABPopt-guided patient's brain perfusion management, to maintain the best achievable intact CA-condition and to protect the patient from brain damage.

**Effects of the invention.** The present invention enables to start CPPopt-guided (or ABPopt-guided) personalized brain perfusion management after measurements not exceeding 3-4 snapshots of CA-status estimation. The CA-status estimate can be obtained and perfusion management decisions taken within a time interval of close to 10 minutes. Such reduced time in making decisions on patients' brain perfusion personalized management means a much shorter delay and more effective decisions, compared to existing delays in intensive care units.

This solution could be efficiently applied in ICUs, for human brain perfusion personalized management during care of TBI, SAH, and ICH patients.

BRIEF DESCRIPTION OF DRAWINGS

The features and advantages of the invention are described in the detailed description of the invention with reference to the drawings below:

**Figure 1** depicts a schematic diagram showing the structure of the embodiment of the apparatus and system for human brain cerebral blood flow autoregulation status identification and personalized brain perfusion management.

**Figure 2** depicts the system operation algorithm showing the steps of generating ABP changes by applying inflatable pants, algorithm for CA identification, and data processing sub-system according to an embodiment of the present invention.

**Figure 3** depicts a chart showing the pants inflation and deflation cycles with different inflation pressures Pe1, Pe2, and the corresponding generation of arterial blood pressure changes according to an embodiment of the present invention.

**Figure 4** is an example of experimentally generated arterial blood pressure changes by applying inflatable pants with applied periodical inflation cycles with different pressures Pe1, Pe2, Pe3.

**Figure 5** depicts an example of a transient response of cerebral blood flow velocity caused by ABP drop during the pants deflation cycle in the case when CA is intact.

**Figure 6** depicts an example of a transient response of cerebral blood flow velocity caused by ABP drop during the pants deflation cycle in the case when CA is impaired.

**Figure 7**     depicts the distribution of delay time ΔT between BV(t) and ABP(t) during the deflation cycle) among patients' outcome groups and the healthy control group.

**Figure 8**     depicts an example of identified ABPopt-guided regulation for personalized brain perfusion by plotting the ΔT factor in relation to the externally induced mean ABP changes.

TERMS AND ABBREVIATIONS OF THE INVENTION

| | |
|---|---|
| **ABP** | arterial blood pressure; |
| **mABP** | mean-ABP, the arterial blood pressure averaged in a time window of a continuous ABP measurement; to obtain mABP, the measured ABP(t) signal is averaged at the end of the pants inflation cycle, within a time window having the length of at least one heartbeat, and preferably, in the range from 1 to 3 seconds; |
| **CA** | cerebral blood flow autoregulation; CA-function of a human, or healthy individual, or patient; CA-status is the CA functional status, considered normal or abnormal, by estimated values of some identified CA-function parameters; |
| **CPP** | cerebral perfusion pressure; |
| **ABPopt** | optimal arterial blood pressure; |
| **CPPopt** | optimal cerebral perfusion pressure; |
| **ICP** | intracranial pressure; |
| **SAH** | subarachnoid hemorrhage; |
| **ICH** | intracerebral hemorrhage; |
| **TCD** | transcranial Doppler; |
| **Pe** | external pressure; |
| **ICU** | intensive care unit; |
| **PRx** | pressure reactivity index; |
| **Mx** | mean flow autoregulation index; |
| **MCA** | middle cerebral artery; |
| **ΔT** | delay between ABP and BV in MCA; |
| **BV** | cerebral blood flow velocity; |
| **POCD** | postoperative cognitive dysfunction. |

DETAILED DESCRIPTION OF THE INVENTION

The invention is a novel method of generating arterial blood pressure (ABP) changes and assessing patients' cerebral blood flow autoregulation (CA) parameters of the human brain. The method is computer-based, i.e., it operates a specifically configured and controlled system, comprising means to generate the external pressure (inflatable pants), to monitor CA transient-response (TCD, Ultrasonic Time-of-Flight (TOF), ICP devices), and computing means for necessary calculations and estimations. The pants inflation subsystem generates a series of periodic inflation-deflation cycles with different inflation pressures Pe1, Pe2,... The period of each such inflation-deflation cycle is 1-3 minutes. At least two cycles with different inflation pressures Pe1, and Pe2 are required to estimate the patient's CA status, and then to provide indications on the raising or lowering patient's mean ABP (mABP) value, for maintaining the best achievable intact CA status.

Further, the system comprises a non-invasive CA monitor (Transcranial Doppler or Ultrasonic Time of Flight) employed to continuously record transient reactions/responses (dynamic autoregulation functions) of the human CA function, those follow the ABP fast changes (drops) induced during the pants deflation cycle.

Since the human brain's CA function is nonlinear, the invention uses monitoring of delay time ΔT between the cerebral blood flow velocity BV and ABP signals during the pants deflation cycle, to reflect the non-linear dynamics of the patient's CA system.

Identification of CA status is performed by calculating the delay time ΔT between the cerebral blood flow velocity BV(t) transient response measured in MCA and the induced change in the ABP(t) signal. Delay time ΔT is estimated as the time difference between minimum points in the BV(t) transient response and the ABP(t) drop signals, induced during the deflation cycle.

An alternative estimation of CA status is possible by calculating the delay time ΔT between intracranial pressure ICP(t) signal and ABP(t) signals. Delay time ΔT is estimated as the time difference between minimum points during the deflation cycle in ICP(t) and ABP(t) signals.

The CA-status estimation according to the measured ΔT is performed at different external inflation pressures (Pe1, Pe2), to collect ΔT data at different mean ABP (mABP) changes.

The collected ΔT data are plotted in relation to the mABP data measured at different Pe cycles (having different pressure peak levels *Pe1* and *Pe2),* to identify the individual's/patient's CA-status, which can further be used for the ABPopt-guided personalized brain perfusion management.

Linear-fitting approximation with slope a and correlation coefficient r between measured ΔT and mABP values are calculated

$$\Delta T = a*mABP + b,$$

for a series of measured ΔT and mABP values:

$$\Delta T(i, i+1, i+2, ...) = a*mABP(i, i+1, i+2, ..) + b,$$

and

$$r = r[\Delta T, mABP].$$

The negative sign of *a* and *r* values provides an indication to medical staff/caregivers for lowering the patient's mABP, for maintaining the best achievable intact CA status, for example, by applying certain therapeutic means, medicaments, etc. The positive sign of *a* and *r* values provides an indication for raising the patient's mABP value, for maintaining the best achievable intact CA status.

The CA-status monitoring system provides information for a caregiving team (including nurses, anesthetists, and surgeons) on the CA status, and correspondingly, regarding the necessity to increase or decrease ABP values to maintain intact CA condition and protect the patient from brain damage within 3-4 inflation cycles, thereby taking up to 10 min to provide an indication for the ABPopt-guided brain perfusion management.

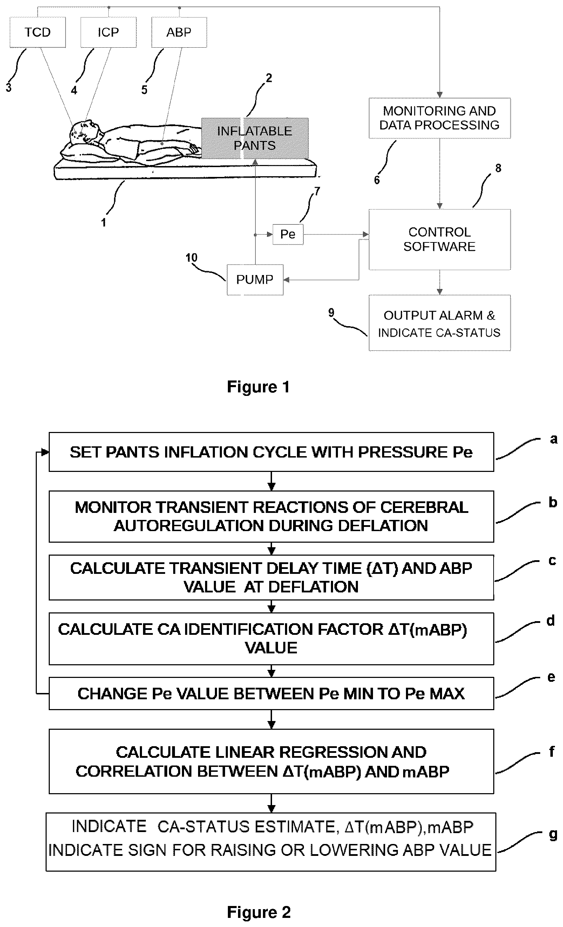

Figure 1 shows the structure of the system embodiment arranged to perform a continuous human brain CA-status monitoring and estimation, and to provide indications to medical staff/caregivers for regarding personalized brain perfusion management.

The system comprises inflatable pants (2) put on human legs, an air pump (10), an inflation pressure Pe measurement unit (7), means of cerebral autoregulation monitoring (any of monitor devices TCD (3), ICP (4), ABP (5)), a monitoring and data processing unit (6), a control unit (8), and a CA-status indicating unit (9). The CA-status indicating unit (9) may provide CA-status indications by different ways: alarming, recording perdiodical CA-status estimates into memory or printing on paper, displaying CA-status on a display or by light indicators, etc.

The inflatable pants (2) are put on human legs (calf and thigh) to apply the external pressure Pe onto the patient's legs, thereby, inducing the corresponding changes to the patient's ABP. The air pump (10) is used to inflate the pants (2) with the external pressure Pe and to form inflation-deflation cycles by inflating the pants (2) up to the external pressure levels Pe1 or Pe2 during the inflation cycle, and releasing pressure back to the baseline Pe0 during the deflation cycle. A Pe-measurement unit (7) is used to control the externally applied pressures. The period of each inflation-deflation cycle is 1-3 min.

Cerebral autoregulation monitoring is performed by using a non-invasive Ultrasonic Transcranial Doppler (TCD) device (3) which monitors cerebral blood flow velocity BV in the middle cerebral artery (MCA) and an invasive (or non-invasive) arterial blood flow monitoring units.

The monitoring and data processing unit (6) collects high-resolution monitoring data and processes it to estimate the delay ΔT between BV (or ICP) and ABP signal drop during deflation. Raw BV (or ICP), and ABP signals are processed to match them in time, according to variations in heart-beat periods.

The control unit (7) is used to control the inflation pressure Pe, and it collects the CA and ABP monitoring data and then processes them to get CA-related measure - ΔT (delay between BV(t) and ABP(t) during the deflation cycle).

The ΔT and mABP data are collected for every deflation cycle measured at inflation pressures of different peak levels Pe1, Pe2, etc. The system analyzes the collected ΔT and mABP data and, on their basis, further provides indications (such as sound alarms, records, light indications) regarding the necessity to raise or lower ABP values, to maintain the best achievable intact CA status.

The schematic diagram in Figure 2 shows the method steps **a-g** of generating ABP changes by the inflatable pants sub-system (2, 7, 10), registering signals by the CA monitoring subsystem (3, 4, 5), and carrying out the algorithm for the CA-status assessment, implemented in the data processing sub-system (6, 8, 9) according to a system embodiment of the present invention, depicted in Figure 1.

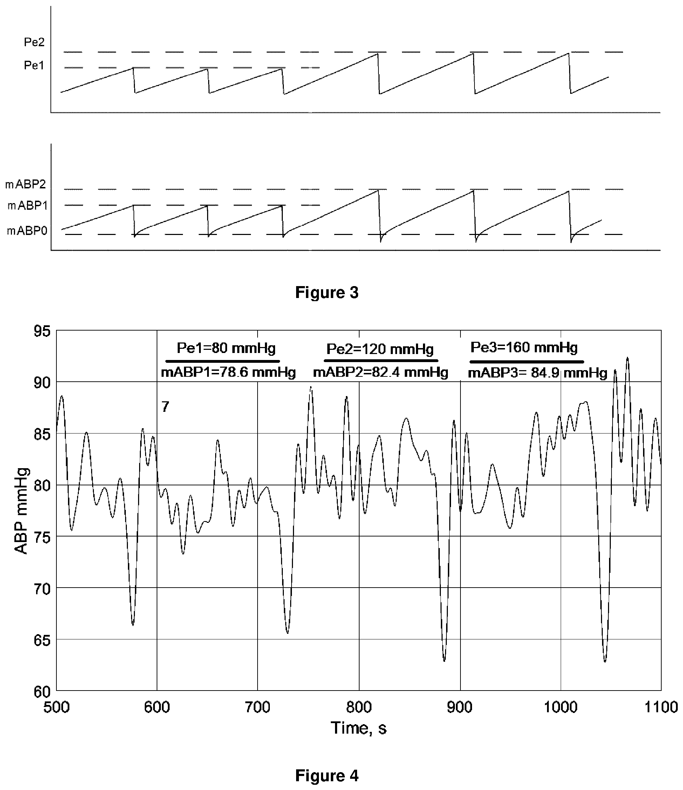

Figure 3 shows the pants inflation and deflation cycles with different inflation pressure peak-levels Pe1, Pe2, ... and the corresponding slow increase of arterial blood pressure during the inflation cycle, and the quick drop during the deflation cycle. The pants inflation cycle takes 1-3 minutes depending on the air pump inflation rate to reach the required external pressure (Pe1, Pe2, ...) applied on the patient's legs (calf and thigh). Pants deflation (air releasing from the pants) takes ~10 sec to get the corresponding quick drop in the ABP(t).

The period of inflating the pants (the inflation cycle) has to be equal to or greater than the shortest physiological slow intracranial blood wave which is approximately 30 seconds. The patient's CA-function has some inertia and a delay in response time for slow ABP changes. The response time of a healthy patient's CA-function is up to 5 seconds [7].

Figure 4 presents an example of experimentally generated ABP changes by means of the inflatable pants and with applied periodical inflation-deflation cycles with different peak-level pressures Pe1, Pe2, Pe3. This example shows that inflating the pants with an external pressure Pe = Pe1 = 80mmHg allows achieving inflation-induced ABP variations with averaged ABP value mABP = 78.6 mmHg and the ABP drops of ~ -10 mmHg during the deflation cycle. By setting the inflation pressure Pe to Pe2=120 mmHg we can increase averaged ABP value to mABP = 82.4 mmHg and get the ABP drop changes ~ -15mmHg. By setting inflation pressure to Pe2=160 mmHg we can increase averaged ABP value to mABP = 84.9 mmHg and get the ABP drop changes ~ -20mmHg.

Inflation-deflation cycles with different controlled external pressure Pe peak-levels Pe1, Pe2, Pe3 ... are applied to achieve mean-ABP increments to corresponding mABP1, mABP2, mABP3 ... levels, which are above baseline value mABP0. The higher the applied external pressure Pe peak-value for pants inflation, the higher mABP changes will be achieved. The applied external pressure Pe values are controlled within safe limits of 80-220 mmHg which are enough to generate a slow mABP increase during the inflation cycle and a fast ABP drop per ~10-20 mmHg during the deflation cycle.

Pants inflation pressure Pe peak-values should be selected within safe limits up to Pe=220 mmHg which is available in most commercially available lymphatic drainage therapy (pressotherapy). Different Pe pressure peak-values Pe1, Pe2, ... should be selected to induce ABP changes up to 5-10 mmHg above the ABP baseline ABP0.

A quick air release from the pants during the deflation cycle induces a correspondingly acute drop in the ABP. This fast drop in ABP activates arterial baroreceptors which via the sympathetic nervous system induce heart rate changes as well as both peripheral and cerebral vasculature changes to recover the cerebral blood flow [6]. At the beginning of the pants deflation, the cerebral blood flow is dropping together with the ABP drop (following it). But, in the presence of intact CA-function, the cerebral blood flow stops decreasing when arterial baroreceptors are triggered due to the ABP drop and starts increasing again before the ABP reaches its minimum value during the deflation cycle. The deflation cycle should be fast enough to induce said acute ABP drop and to stimulate reactions of the the CA functions, and slow enough to cover the whole autoregulatory reaction in cerebral blood flow before the ABP drop reaches its minimum value during the deflation cycle. Therefore, the optimal range of the pants deflation cycle duration should be within the range of 5 to 15 seconds to register the BV response time before the ABP value drops to the minimum point after the pants deflation.

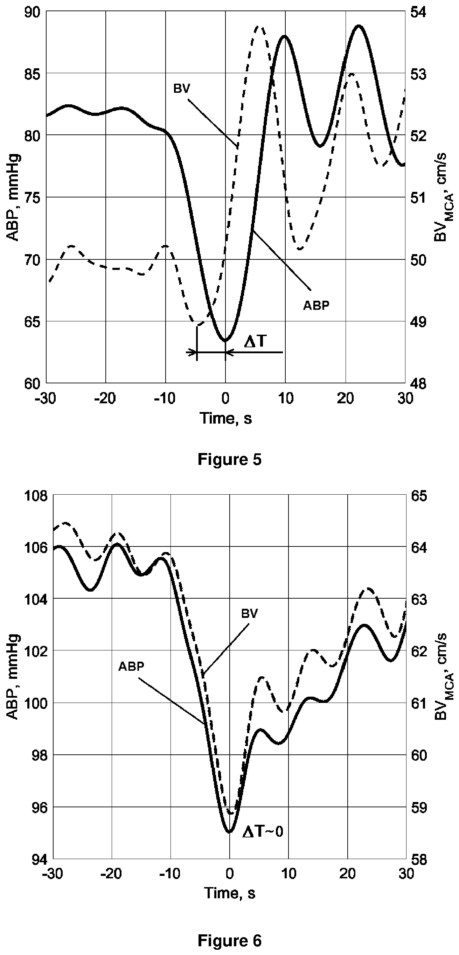

Figure 5 shows the transient response of a human CA function during the deflation cycle in the case of intact autoregulation by the CA-function. The cerebral blood flow velocity (BV) signal exceeds the ABP signal before the ABP signal reaches the minimum point during the deflation cycle due to the activity of the CA function. A normal delay time ΔT between the BV and ABP signals minimum points should be within ~2-5 seconds.

Figure 6 shows the transient response of a human CA-function during the deflation cycle in the case of impaired autoregulation. The minimal delay between cerebral blood flow velocity BV signal and ABP signal is observed due to some impairments of the CA-function. The delay time between BV and ABP signals is close to zero (less than 1.6) seconds in the case of impaired CA-function.

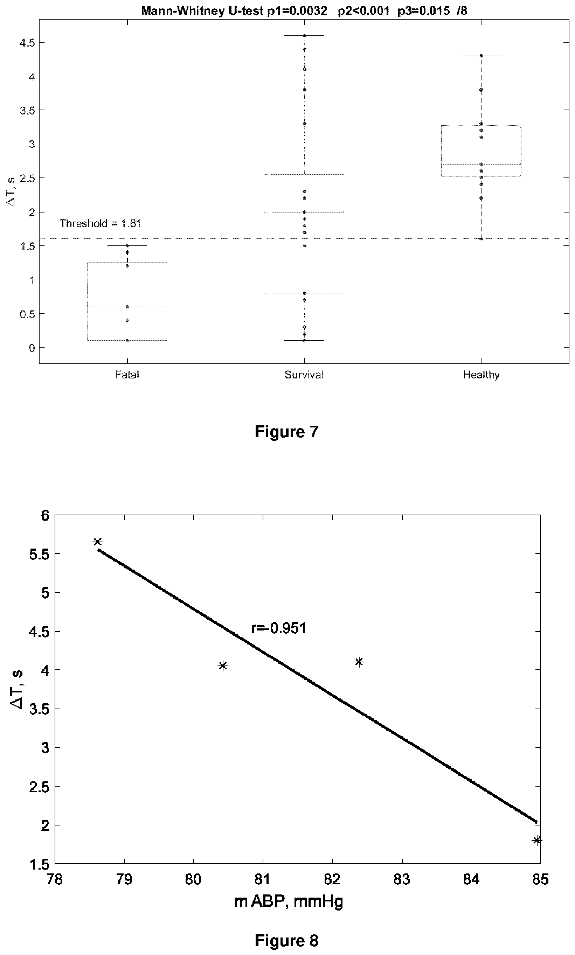

Figure 7 shows the distribution of delay time ΔT between ABP(t) and BV(t) during the pants deflation cycle, identified for SAH and ICH patients with fatal and survival outcome groups and the healthy control group.

Figure 8 shows the case of identifying information regarding the necessary ABP regulation according to the factor ΔT to keep the best achievable CA status. The ΔT and mABP values were identified for each deflation cycle. Linear regression curves between ΔT and mABP show the direction needed for increasing or decreasing ABP value to maintain the best achievable intact CA.

Figure 8 shows an example when the obtained negative correlation coefficient r<0 shows that mABP should be kept lower than actual ABP values to maintain brain perfusion by the best achievable intact CA-function.

Non-invasive cerebral autoregulation monitoring can be based on Transcranial Doppler (TCD), Ultrasonic Time of Flight (TOF) technologies, near-infrared spectroscopy (NIRS) technologies, or other CA monitoring technologies with sub-second temporal resolution.

Invasive CA monitoring can be based on direct intracranial pressure (ICP) monitoring technologies providing a sub-second temporal resolution. The CA-monitoring system can be connected to a separately installed ICP monitor, for accessing the ICP monitoring data and use this data for CA-status estimation.

Based on the obtained indications on CA-status and necessity of regulating the patient's ABP, the caregivers can take corresponding therapies and therapeutic means to change mABP value, thereby optimizing the patient's brain perfusion and maintaining the best achievable intact CA-status. Note, that therapies and therepeutic means to change mABP value are different means from those providing an external pressure Pe in the present invention to estimate the CA-status (such as inflatable pants).

Typically, the CA status is identified to be intact if the estimated $\Delta T$ value is within the 2-5 seconds range. Nevertheless, the normal $\Delta T$ ranges are individual for each person. Lower $\Delta T$ values should be avoided, therefore, ABP regulation should be performed by increasing or decreasing the mABP, to keep $\Delta T$ at the highest achieved value for a person.

Other metrics derived from ABP(t) and BV(t) signals and their changes during the deflation cycle could also be used as additional CA-related factors (i.e., correlation coefficients between ABP(t) and BV(t) signals during the deflation cycle, $\Delta T$ value divided by the ABP-drop amplitude, etc.) and applied for ABPopt-guided personalized brain perfusion management.

In the case of invasive ICP monitoring, the collected $\Delta T$ values are plotted in relation to the mean-CPP values during the deflation cycle and further can be used for CPPopt-guided personalized brain perfusion management. Mean-CPP values calculated from the measured mean-ABP and mean-ICP values as:

$$\text{Mean CPP} = \text{mean ABP} - \text{mean ICP}.$$

The CA status obtained estimates should be verified in a certain order, during the diagnostic procedure by medical staff. For example, when employing $\Delta T$ and a, r parameters to provide an indication to medical staff for ABP management:

- first, the $\Delta T$ value should be checked, whether it falls into the normal range (2 to 5 seconds), and only then if not - an indication that the ABP has to be regulated, therefore, then
- **a** and **r** parameters are estimated, and negative or positive signs of the **a/r** values should be checked, providing a further (second) indication, for whether the patient's ABP has to be raised or lowered, by some therapeutic means.

A practical embodiment of the invented system and method, for example, implemented in an ICU, may comprise a corresponding configured equipment attached to a patient, and monitoring his CA-status, whereby periodically recording, e.g., every 10 minutes, estimated values of the CA-status variables:

- $\Delta T$, with a provided indication (e.g., on a display, or light indicators) of whether it is normal, too low, or too high;
- **a** and **r**, with a negative or positive sign, and with an indication (e.g., on a display) to medical staff for raising or lowering the patient's ABP.

During such continuous monitoring, a system alarm may be configured for an event, when the $\Delta\mathbf{T}$ estimates begin to fall out from the normal range, and the **a** and **r** estimates show corresponding indications to take ABP management actions. Another option: a medical staff member (doctor) reviews periodically the recorded history of the CA-estimates, and may decide whether some ABP and brain perfusion management is necessary for the patient.

One more possible option is how the ABP regulation using therapeutic means can be performed:

- in the case of $\Delta T > 3$ seconds, the system providing an indication, for intact CA-status and no mABP regulation;
- in case of $\Delta T < 3$ seconds and $\Delta T > 1.6$ seconds, the system providing an indication, for the CA-status and mABP regulation by therapeutic means, to achieve the maximum possible $\Delta T$ value or the mABP value at which the $\Delta T$ is the maximal possible; and
- in case of $\Delta T < 1.6$ seconds, the system providing an indication, for the CA-status and mABP continuous regulation by therapeutic means, until at least $\Delta T = 1.6$ seconds value is reached.

Although this invention has been shown and described for the detailed embodiments thereof, it will be understood by those of skill in the art that various changes may be made and equivalents may be substituted for elements thereof without departing from the scope of the invention. In addition, modifications may be made to adapt a particular situation or material to the teaching of the invention without departing from the essential scope thereof. Therefore, it is intended that the invention not be limited to the particular embodiments disclosed in the detailed description, but that the invention will include all embodiments falling within the scope of the appended claims.

REFERENCES

**[1]** Petkus V, Preiksaitis A, Chaleckas E, Chomskis R, Zubaviciute E, Vosylius S, Rocka S, Rastenyte D, Aries MJ, Ragauskas A, Neumann JO. Optimal Cerebral Perfusion Pressure: Targeted Treatment for Severe Traumatic Brain Injury. J Neurotrauma. 2020 Jan 15;37(2):389-396. doi: 10.1089/neu.2019.6551. Epub 2019 Nov 13. PMID:

31583962.

[2] Kumpaitiene B, Svagzdiene M, Sirvinskas E, Adomaitiene V, Petkus V, Zakelis R, Krakauskaite S, Chomskis R, Ragauskas A, Benetis R. Cerebrovascular autoregulation impairments during cardiac surgery with cardiopulmonary bypass are related to postoperative cognitive deterioration: prospective observational study. Minerva Anestesiol. 2019 Jun;85(6):594-603. doi: 10.23736/S0375-9393.18.12358-3. Epub 2018 May 11. PMID: 29756691.

[3] Deimantavicius M, Chaleckas E, Boere K, Putnynaite V, Tamosuitis T, Tamasauskas A, Kavaliauskas M, Rocka S, Preiksaitis A, Vosylius S, Krakauskaite S, Berskiene K, Petkus V, Ragauskas A. Feasibility of the optimal cerebral perfusion pressure value identification without a delay that is too long. Sci Rep. 2022 Oct 22;12(1):17724. doi: 10.1038/s41598-022-22566-6. PMID: 36272984; PMCID: PMC9588030.

[4] Steiner LA, et al. Continuous monitoring of cerebrovascular pressure reactivity allows determination of optimal cerebral perfusion pressure in patients with traumatic brain injury. Crit. Care Med. 2002;30:733-738. doi: 10.1097/00003246-200204000-00002. - DOI - PubMed

[5] Olsen MH, Riberholt CG, Mehlsen J, Berg RM, Møller K. Reliability and validity of the mean flow index (Mx) for assessing cerebral autoregulation in humans: A systematic review of the methodology. J Cereb Blood Flow Metab. 2022 Jan;42(1):27-38. doi: 10.1177/0271678X211052588. Epub 2021 Oct 7. PMID: 34617816; PMCID: PMC8721771.

[6] Ogoh, S., Tarumi, T. Cerebral blood flow regulation and cognitive function: a role of arterial baroreflex function. J Physiol Sci 69, 813-823 (2019). https://doi.org/10.1007/s12576-019-00704-6

[7] Simpson DM, Payne SJ, Panerai RB. The INfoMATAS project: Methods for assessing cerebral autoregulation in stroke. Journal of Cerebral Blood Flow & Metabolism. 2022;42(3):411-429. doi:10.1177/0271678X211029049

[8] RAGAUSKAS ARMINAS [LT]; PETKUS VYTAUTAS [LT]; CHALECKAS EDVINAS [LT], METHOD AND APPARATUS FOR HUMAN BRAIN NEUROPROTECTION DURING SURGERY, US2023109678 (A1), 2023-04-13. [9] AASLID R ET AL. Cerebral autoregulation dynamics in humans. STROKE, vol. 20, no. 11 January 1989 (1989-01-01), pages 45-52.

## Claims

1. A method of estimating a human cerebrovascular blood flow autoregulation status (CA-status), **comprising** steps of

    • **(a)** applying cyclic inflations-deflations (7) by a controlled external pressure $Pe$ means applied to a patient's body (1), thereby inducing the arterial blood pressure (ABP) waves and drop steps,

       o wherein **(e)** at least two inflation-deflation cycles with different inflation pressure peak levels $Pe1, Pe2$ are applied; and simultaneously

    • **(b)** recording monitored transient responses as dynamic functions of the CA-status, by non-invasive CA-response-monitoring means (3, 5); and further
    • estimating the CA-status from said transient responses recorded during the external pressure $Pe$ deflation cycles, wherein

       o **(c)** a delay time $\Delta T$ is estimated as a time difference between the minimum points in the CA-transient-response and the ABP(t) signal, for each of the at least two inflation-deflation cycles with the different pressure peak-levels $Pe1, Pe2$;
       o **(d)** estimating a CA-status factor $\Delta T(mABP)$ for said at least two inflation-deflation cycles, wherein the mABP is a mean of the ABP(t) signal averaged within a time-window having a length of at least one heartbeat at the end of each said $Pe1, Pe2$ inflation cycles;
       o **(f)** estimating the CA-status comprising values $a$ as a linear regression coeficient of $\Delta T = a*mABP + b$, and r as a correlation coefficient $r=r[\Delta T, mABP]$, from the $\Delta T(mABP)$ values at said least two inflation-deflation cycles;

    • (g) output or indicating the estimated CA-status .

2. The method according to claim 1, **wherein** the mABP is a mean of the ABP(t) signal averaged within a time-window having a length in the range from 1 to 3 seconds, at the end of the external pressure inflation cycle and before the external pressure deflation cycle.

3. The method of claim 1, **wherein** the external pressure $Pe$ cyclic inflation-deflation cycles are generated using inflatable pants (2), an air pump (10), and an external pressure control system (7), by inflating the pants (2) with said controlled external pressure **Pe.**

4. The method of claim 1, **wherein** the CA-monitoring-means is a non-invasive CA monitor, any one of Transcranial Doppler (3), or Ultrasonic Time of Flight monitors, or near-infrared spectroscopy (NIRS) monitors, having a sub-second temporal resolution, continuously recording the CA-transient-responses, wherein

   • the CA-transient-response time is estimated as the delay time $\Delta T$ between a cerebral blood flow velocity BV(t) measured in the middle cerebral artery (MCA), and the ABP(t).

5. The method of claim 1, **wherein** said external pressure **Pe** cyclic inflations-deflations, applied for changing the patient's ABP, have:

   • the inflation cycle period is 1-3 minutes;
   • the deflation cycle period is 5-15 seconds.

6. The method of claim 1, **wherein** said external pressure **Pe** cyclic inflations-deflations, applied for changing the patient's ABP, have:

   • controlled **Pe** values within the safe range of 80-220mmHg, for a slow mABP, increase up to 5-10mmHg above the mABP baseline during the inflation cycle and a faster mABP drop of 10-20mmHg during the deflation cycle.

7. The method of claim 1, **wherein** in a single measurement of the CA-status, at least two inflation-deflation cycles with the different pressure levels **Pe1, Pe2,** each one is repeated, preferably, from 2 to 4 times, and the estimated $\Delta T$ and mABP values are averaged within each group of the repeated cycles.

8. A system for estimating a patient's CA-status, comprising at least

   • inflatable pants (2) with an air pump (10), and external pressure control system (7), arranged to apply a controlled external pressure **Pe** cyclic inflations-deflations (7) to the patient's body (1);
   • CA-response-monitoring means (3,4,5), recording transient responses, as dynamic functions of the patient's CA status;
   • monitoring and data processing component (6), system control component (8), and alarm-indication components (9),

   **wherein** the system is configured to:

   • **(a)** apply cyclic inflations-deflations (7) of the controlled external pressure **Pe** to a patient's body (1), thereby inducing the arterial blood pressure (ABP) waves and drop steps,

      o wherein **(e)** at least two inflation-deflation cycles with different inflation pressure peak levels **Pe1, Pe2** are applied; and simultaneously

   • **(b)** record monitored transient responses as dynamic functions of the CA-status, by the CA-response-monitoring means (3,4,5); and further
   • estimating the CA-status from said transient responses recorded during the external pressure **Pe** deflation cycles, wherein

      o **(c)** a delay time $\Delta T$ is estimated as a time difference between the minimum points in the CA-transient-response and the ABP(t) signal, for each of the at least two inflation-deflation cycles with the different pressure peak-levels **Pe1, Pe2;**
      o **(d)** estimating a CA-status factor $\Delta T(mABP)$ for said at least two inflation-deflation cycles, wherein the mABP is a mean of the ABP(t) signal averaged within a time-window having a length of at least one heartbeat at the end of each said **Pe1, Pe2** inflation cycles
      o **(f)** estimating the CA-status comprising values **a** as a linear regression coeficient of $\Delta T = a*mABP + b,$ and **r** as a correlation coefficient **r=r[$\Delta T$, mABP],** from the $\Delta T(mABP)$ values at said least two inflation-deflation cycles;

• (g) output the estimated CA-status .

9. The system according to claim 8,**wherein** the CA-monitoring-means for continuous recording of the CA-transient-responses is any one of CA monitors having a sub-second temporal resolution:

• a non-invasive CA monitor, such as Transcranial Doppler (3), or Ultrasonic Time of Flight monitor, or near-infrared spectroscopy (NIRS) monitor;
• or/and is connected to record monitoring data from an invasive intracranial pressure (ICP) monitor (4).

10. The system according to claim 8, **wherein** the mABP is a mean of the ABP(t) signal averaged within a time-window having a length in the range from 1 to 3 seconds, at the end of the external pressure inflation cycle and before the external pressure deflation cycle.

11. The system according to claim 8, **wherein** said external pressure *Pe* cyclic inflations-deflations, applied for changing the patient's ABP, have:

• the inflation cycle period is 1-3 minutes;
• the deflation cycle period is 5-15 seconds.

12. The system according to claim 8, **wherein** said external pressure *Pe* cyclic inflations-deflations, applied for changing the patient's ABP, have:

• controlled *Pe* values within the safe range of 80-220mmHg, for a slow mABP, increase up to 5-10mmHg above the mABP baseline during the inflation cycle and a faster mABP drop of 10-20mmHg during the deflation cycle.

13. The system according to claim 8, **wherein** in a single measurement of the CA-status, at least two inflation-deflation cycles with the different pressure levels *Pe1, Pe2,* each one is repeated, preferably, from 2 to 4 times, and the estimated $\Delta T$ and mABP values are averaged within each group of the repeated cycles.

14. The system according to claim 8, **wherein** the system indicates the CA-status as follows:

a) if the transient response delay $\Delta T$ is within 2 to to 5 seconds range or more, the system indicates intact CA-function; and
**b)** if the transient response delay $\Delta T$ is less than 1.6 seconds, the system indicates an abnormal CA-function, and further based on the *r* and **a** estimated values sign, wherein

• the *r* and **a** sign is negative, the system indicates for lowering the ABP value;
• the *r* and **a** sign are positive, the system indicates for raising the ABP value.

15. The system according to claim 14, **wherein** the system further indicates the CA-status as follows:

• in case of $\Delta T>3$ seconds, no mABP regulation required;
• in case of $\Delta T<3$ seconds and $\Delta T > 1.6$ seconds, for the CA-status and mABP regulation to achieve the maximum possible $\Delta T$ value, or the mABP value at which the $\Delta T$ is the maximal possible; and
• in case of $\Delta T<1.6$ seconds, for the CA-status and mABP continuous regulation until at least $\Delta T=1.6$ seconds value is achieved.

**Patentansprüche**

1. Verfahren zum Schätzen eines Status der humanen zerebrovaskulären Blutflussautoregulierung (CA-Status), das die Schritte umfasst:

• **(a)** Anwenden von zyklischen Aufblas- und Entleerungsvorgängen (7) durch ein Mittel für kontrollierten externen Druck *Pe,* das auf einen Körper (1) eines Patienten angewandt wird, wodurch die Wellen und Abfallstufen des arteriellen Blutdrucks (ABP) induziert werden,

o wobei **(e)** zumindest zwei Aufblasen-Entleeren-Zyklen mit unterschiedlichen Aufblasdruckspitzenhöhen

*Pe1, Pe2* angewandt werden; und gleichzeitig

• **(b)** Aufzeichnen von überwachten transienten Antworten als dynamische Funktionen des CA-Status durch ein nichtinvasives CA-Antwort-Überwachungsmittel (3, 5); und ferner
• Schätzen des CA-Status anhand der transienten Antworten, die während der Entleerungszyklen mit externem Druck **Pe** aufgezeichnet werden, wobei

o **(c)** eine Verzögerungszeit $\Delta T$ als Zeitunterschied zwischen den Mindestpunkten in der transienten CA-Antwort und dem ABP(t)Signal für jeden der zumindest zwei Aufblasen-Entleeren-Zyklen mit den unterschiedlichen Druckspitzenhöhen *Pe1, Pe2* geschätzt wird;
o **(d)** Schätzen eines CA-Statusfaktors $\Delta T(mABP)$ für die zumindest zwei Aufblasen-Entleeren-Zyklen, wobei der mABP ein Mittelwert des ABP(t)-Signals ist, dessen Durchschnitt innerhalb eines Zeitfensters mit einer Länge von zumindest einem Herzschlag am Ende jedes *Pe1-, Pe2*-Aufblaszyklus ermittelt wurde;
o **(f)** Schätzen des CA-Status, umfassend Werte **a** als linearer Regressionskoeffizient von $\Delta T = a * mABP + b$ **und r** als Korrelationskoeffizient $r = r[\Delta T, mABP]$ anhand der $\Delta T(mABP)$-Werte der zumindest zwei Aufblasen-Entleeren-Zyklen;

• **(g)** Ausgeben oder Angeben des geschätzten CA-Status.

2. Verfahren nach Anspruch 1, wobei der mABP ein Mittelwert des ABP(t)-Signals ist, dessen Durchschnitt innerhalb eines Zeitfensters mit einer Länge in dem Bereich von 1 bis 3 Sekunden am Ende des Aufblaszyklus mit externem Druck und vor dem Entleerungszyklus mit externem Druck ermittelt wurde.

3. Verfahren nach Anspruch 1, wobei die zyklischen Aufblasen-Entleeren-Zyklen mit externem Druck **Pe** unter Verwendung einer aufblasbaren Hose (2), einer Luftpumpe (10) und eines Steuersystems (7) für externen Druck durch Aufblasen der Hose (2) mit dem kontrollierten externen Druck **Pe** erzeugt werden.

4. Verfahren nach Anspruch 1, wobei das CA-Überwachungsmittel ein nichtinvasiver CA-Monitor, eines von transkraniellen Doppler-Monitoren (3) oder Ultraschall-Time-of-Flight-Monitoren oder Nahinfrarot-Spektroskopie-(NIRS-) Monitoren mit einer zeitlichen Auflösung im Subsekundenbereich ist, der die transienten CA-Antworten kontinuierlich aufzeichnet, wobei

• die transiente CA-Antwortzeit als Verzögerungszeit $\Delta T$ zwischen einer zerebralen Blutflussgeschwindigkeit BV(t), die in der mittleren Hirnschlagader (MCA) gemessen wird, und dem ABP(t) geschätzt wird.

5. Verfahren nach Anspruch 1, wobei die zyklischen Aufblas- und Entleerungsvorgänge mit externem Druck *Pe,* die zum Ändern des ABP des Patienten angewandt werden, aufweisen:

• die Zeitdauer eines Aufblaszyklus beträgt 1 bis 3 Minuten;
• die Zeitdauer eines Entleerungszyklus beträgt 5 bis 15 Sekunden.

6. Verfahren nach Anspruch 1, wobei die zyklischen Aufblas- und Entleerungsvorgänge mit externem Druck *Pe,* die zum Ändern des ABP des Patienten angewandt werden, aufweisen:

• kontrollierte *Pe*-Werte innerhalb des sicheren Bereichs von 80 bis 220 mmHg für einen langsamen mABP, Erhöhung auf bis zu 5 bis 10 mmHg über der mABP-Baseline während des Aufblaszyklus und einen schnelleren mABP-Abfall von 10 bis 20 mmHg während des Entleerungszyklus.

7. Verfahren nach Anspruch 1, wobei bei einer einzelnen Messung des CA-Status zumindest zwei Aufblasen-Entleeren-Zyklen mit den unterschiedlichen Druckhöhen *Pe1, Pe2* erfolgen, wobei jeder davon wiederholt wird, vorzugsweise 2- bis 4-mal, und der Durchschnitt der geschätzten $\Delta T$- und mABP-Werte innerhalb jeder Gruppe der wiederholten Zyklen ermittelt wird.

8. System zum Schätzen eines CA-Status eines Patienten, das zumindest umfasst

• eine aufblasbare Hose (2) mit einer Luftpumpe (10) und einem Steuersystem (7) für externen Druck, dazu ausgelegt, zyklische Aufblas- und Entleerungsvorgänge (7) mit einem gesteuerten externen Druck *Pe* auf den Körper (1) des Patienten anzuwenden;

• ein CA-Antwort-Überwachungsmittel (3, 4, 5), das transiente Antworten als dynamische Funktionen des CA-Status des Patienten aufzeichnet;
• eine Überwachungs- und Datenverarbeitungskomponente (6), eine Systemsteuerkomponente (8) und Alarmausgabekomponenten (9),

wobei das System dazu konfiguriert ist:

• **(a)** Anwenden von zyklischen Aufblas- und Entleerungsvorgängen (7) des kontrollierten externen Drucks **Pe** auf einen Körper (1) eines Patienten, wodurch die Wellen und Abfallstufen des arteriellen Blutdrucks (ABP) induziert werden,

o wobei **(e)** zumindest zwei Aufblasen-Entleeren-Zyklen mit unterschiedlichen Aufblasdruckspitzenhöhen **Pe1, Pe2** angewandt werden; und gleichzeitig

• **(b)** Aufzeichnen von überwachten transienten Antworten als dynamische Funktionen des CA-Status durch das CA-Antwort-Überwachungsmittel (3, 4, 5); und ferner
• Schätzen des CA-Status anhand der transienten Antworten, die während der Entleerungszyklen mit externem Druck **Pe** aufgezeichnet werden, wobei

o (**c**) eine Verzögerungszeit $\Delta T$ als Zeitunterschied zwischen den Mindestpunkten in der transienten CA-Antwort und dem ABP(t)Signal für jeden der zumindest zwei Aufblasen-Entleeren-Zyklen mit den unterschiedlichen Druckspitzenhöhen **Pe1, Pe2** geschätzt wird;
o **(d)** Schätzen eines CA-Statusfaktors $\Delta T(mABP)$ für die zumindest zwei Aufblasen-Entleeren-Zyklen, wobei der mABP ein Mittelwert des ABP(t)-Signals ist, dessen Durchschnitt innerhalb eines Zeitfensters mit einer Länge von zumindest einem Herzschlag am Ende jedes **Pe1-, Pe2**-Aufblaszyklus ermittelt wurde;
o **(f)** Schätzen des CA-Status, umfassend Werte **a** als linearer Regressionskoeffizient von $\Delta T = a * mABP + b$ und **r** als Korrelationskoeffizient **r = r[$\Delta$T, mABP]** anhand der $\Delta T(mABP)$-Werte der zumindest zwei Aufblasen-Entleeren-Zyklen;

• **(g)** Ausgeben des geschätzten CA-Status.

9.  System nach Anspruch 8, wobei das CA-Überwachungsmittel zur kontinuierlichen Aufzeichnung der transienten CA-Antworten eines von CA-Monitoren mit einer zeitlichen Auflösung im Subsekundenbereich ist:

• ein nichtinvasiver CA-Monitor wie ein transkranieller Doppler-Monitor (3) oder Ultraschall-Time-of-Flight-Monitor oder Nahinfrarot-Spektroskopie-(NIRS-)Monitor;
• und/oder verbunden ist, um Überwachungsdaten von einem invasiven Monitor (4) für intrakraniellen Druck (ICP) aufzuzeichnen.

10. System nach Anspruch 8, wobei der mABP ein Mittelwert des ABP(t)-Signals ist, dessen Durchschnitt innerhalb eines Zeitfensters mit einer Länge in dem Bereich von 1 bis 3 Sekunden am Ende des Aufblaszyklus mit externem Druck und vor dem Entleerungszyklus mit externem Druck ermittelt wurde.

11. System nach Anspruch 8, wobei die zyklischen Aufblas- und Entleerungsvorgänge mit externem Druck **Pe**, die zum Ändern des ABP des Patienten angewandt werden, aufweisen:

• die Zeitdauer eines Aufblaszyklus beträgt 1 bis 3 Minuten;
• die Zeitdauer eines Entleerungszyklus beträgt 5 bis 15 Sekunden.

12. System nach Anspruch 8, wobei die zyklischen Aufblas- und Entleerungsvorgänge mit externem Druck **Pe**, die zum Ändern des ABP des Patienten angewandt werden, aufweisen:

• kontrollierte **Pe**-Werte innerhalb des sicheren Bereichs von 80 bis 220 mmHg für einen langsamen mABP, Erhöhung auf bis zu 5 bis 10 mmHg über der mABP-Baseline während des Aufblaszyklus und einen schnelleren mABP-Abfall von 10 bis 20 mmHg während des Entleerungszyklus.

13. System nach Anspruch 8, wobei bei einer einzelnen Messung des CA-Status zumindest zwei Aufblasen-Entleeren-Zyklen mit den unterschiedlichen Druckhöhen **Pe1, Pe2** erfolgen, wobei jeder davon wiederholt wird, vorzugsweise 2- bis 4-mal, und der Durchschnitt der geschätzten ∆T- und mABP-Werte innerhalb jeder Gruppe der wiederholten Zyklen ermittelt wird.

**14.** System nach Anspruch 8, wobei das System den CA-Status wie folgt angibt:

a) wenn die transiente Antwortverzögerung∆T innerhalb eines Bereichs von 2 bis 5 Sekunden oder mehr liegt, gibt das System eine intakte CA-Funktion an; und
b) wenn die transiente Antwortverzögerung∆T weniger als 1,6 Sekunden beträgt, zeigt das System eine anomale CA-Funktion an, und ferner auf der Grundlage des geschätzten *r*- und *a*-Wertevorzeichens, wobei:

• wenn das *r*- und *a*-Zeichen negativ ist, das System eine Senkung des ABP-Werts angibt;
• wenn das *r*- und *a*-Zeichen positiv ist, das System eine Erhöhung des ABP-Werts angibt.

**15.** System nach Anspruch 14, wobei das System ferner den CA-Status wie folgt angibt:

• wenn ∆T > 3 Sekunden, ist keine mABP-Regulierung erforderlich;
• wenn ∆T < 3 Sekunden und∆T > 1,6 Sekunden, CA-Status- und mABP-Regulierung, bis der maximal mögliche ∆T-Wert erreicht ist oder der mABP-Wert, bei dem ∆T größtmöglich ist; und
• wenn ∆T < 1,6 Sekunden, kontinuierliche CA-Status- und mABP-Überwachung, bis zumindest ∆T = 1,6 Sekunden erreicht ist.


**Revendications**

**1.** Procédé d'estimation de l'état d'autorégulation du flux sanguin cérébrovasculaire humain (état CA), comprenant les étapes consistant à :

• **(a)** appliquer des gonflements-dégonflements cycliques (7) par un moyen de pression externe contrôlée **Pe** appliquée au corps d'un patient (1), induisant ainsi les ondes et les paliers de chute de la pression artérielle (ABP),

o dans lequel **(e)** au moins deux cycles de gonflage-dégonflage avec différents niveaux de pic de pression de gonflage **Pe1, Pe2** sont appliqués ; et simultanément

• **(b)** enregistrer des réponses transitoires surveillées en tant que fonctions dynamiques de l'état CA, par un moyen de surveillance de réponse CA non invasif (3, 5) ; et en outre
• estimer l'état CA à partir desdites réponses transitoires enregistrées pendant les cycles de dégonflage **Pe** sous pression externe, dans lequel

o **(c)** un temps de retard ∆T est estimé comme une différence de temps entre les points minimaux dans la réponse transitoire CA et le signal ABP(t), pour chacun des au moins deux cycles de gonflage-dégonflage avec les différents niveaux de pic de pression **Pe1, Pe2** ;
o **(d)** l'estimation d'un facteur d'état CA ∆T(mABP) pour lesdits au moins deux cycles de gonflage-dégonflage, dans lequel la mABP est une moyenne du signal ABP(t) moyenné dans une fenêtre temporelle ayant une longueur d'au moins un battement de cœur à la fin de chacun desdits cycles de gonflage **Pe1, Pe2** ;
o **(f)** l'estimation du statut CA comprenant des valeurs a comme coefficient de régression linéaire de **∆T = a\*mABP+ b,** et *r* comme coefficient de corrélation **r= r[∆T, mABP],** à partir des valeurs ∆T (mABP) auxdits au moins deux cycles d'inflation-déflation ;

• (**g**) sortir ou indiquer le statut CA estimé.

**2.** Procédé selon la revendication 1, dans lequel la mABP est une moyenne du signal ABP(t) moyenné dans une fenêtre temporelle ayant une longueur dans la plage de 1 à 3 secondes, à la fin du cycle de gonflage à pression externe et avant le cycle de dégonflage à pression externe.

**3.** Procédé selon la revendication 1, dans lequel les cycles de gonflage-dégonflage cycliques à pression externe **Pe** sont générés en utilisant un pantalon gonflable (2), une pompe à air (10) et un système de contrôle de pression externe (7), en gonflant le pantalon (2) avec ladite pression externe contrôlée **Pe.**

4. Procédé selon la revendication 1, dans lequel le moyen de surveillance de CA est un moniteur de CA non invasif, par exemple un Doppler transcrânien (3), un moniteur à temps de vol ultrasonique ou un moniteur de spectroscopie proche infrarouge (NIRS), ayant une résolution temporelle inférieure à la seconde, enregistrant en continu les réponses transitoires de CA, dans lequel

> • le temps de réponse transitoire de CA est estimé comme le temps de retard $\Delta T$ entre une vitesse du flux sanguin cérébral BV(t) mesurée dans l'artère cérébrale moyenne (MCA) et l'ABP (t) .

5. Procédé selon la revendication 1, dans lequel lesdits gonflages-dégonflages cycliques à pression externe *Pe*, appliqués pour modifier l'ABP du patient, ont :

> • la durée du cycle de gonflage est de 1 à 3 minutes ;
> • la période du cycle de dégonflage est de 5 à 15 secondes.

6. Procédé selon la revendication 1, dans lequel lesdits gonflages-dégonflages cycliques à pression externe *Pe,* appliqués pour modifier l'ABP du patient, ont :

> • des valeurs **Pe** contrôlées dans la plage de sécurité de 80 à 220 mmHg, pour une mABP lente , augmentant jusqu'à 5 à 10 mmHg au-dessus de la ligne de base de la mABP pendant le cycle de gonflage et une chute plus rapide de la mABP de 10 à 20 mmHg pendant le cycle de dégonflage.

7. Procédé selon la revendication 1, dans lequel, dans une mesure unique de l'état CA, au moins deux cycles de gonflage-dégonflage avec les différents niveaux de pression *Pe1, Pe2,* chacun est répété, de préférence, de 2 à 4 fois, et les valeurs estimées $\Delta$de T et de mABP sont moyennées dans chaque groupe des cycles répétés.

8. Système permettant d'estimer le statut CA d'un patient, comprenant au moins

> • un pantalon gonflable (2) avec une pompe à air (10), et un système de contrôle de pression externe (7), agencé pour appliquer une pression externe contrôlée *Pe* de gonflages-dégonflages cycliques (7) au corps du patient (1) ;
> • un moyen de surveillance de la réponse CA (3, 4, 5), enregistrant les réponses transitoires, en tant que fonctions dynamiques de l'état CA du patient ;
> • un composant de surveillance et de traitement des données (6), composant de contrôle du système (8) et composants d'indication d'alarme (9),

dans lequel le système est configuré pour :

> • **(a)** appliquer des gonflages-dégonflages cycliques (7) de la pression externe contrôlée **Pe** au corps d'un patient (1), induisant ainsi les ondes de pression artérielle (ABP) et les paliers de chute,
>
> > o dans lequel **(e)** au moins deux cycles de gonflage-dégonflage avec différents niveaux de pic de pression de gonflage *Pe1, Pe2* sont appliqués ; et simultanément
>
> • **(b)** enregistrer les réponses transitoires surveillées en tant que fonctions dynamiques de l'état CA, par le moyen de surveillance des réponses CA (3, 4, 5) ; et en outre
> • estimer l'état CA à partir desdites réponses transitoires enregistrées pendant les cycles de dégonflage **Pe** sous pression externe, dans lequel
>
> > o **(c)** un temps de retard $\Delta T$ est estimé comme une différence de temps entre les points minimaux dans la réponse transitoire CA et le signal ABP(t), pour chacun des au moins deux cycles de gonflage-dégonflage avec les différents niveaux de pic de pression *Pe1, Pe2* ;
> > o **(d)** l'estimation d'un facteur d'état CA $\Delta$T(mABP) pour lesdits au moins deux cycles de gonflage-dégonflage, dans lequel la mABP est une moyenne du signal ABP(t) moyenné dans une fenêtre temporelle ayant une longueur d'au moins un battement de cœur à la fin de chacun desdits cycles de gonflage *Pe1, Pe2*
> > o **(f)** l'estimation du statut CA comprenant des valeurs **a** comme coefficient de régression linéaire de $\Delta$**T = a\*mABP+b,** et *r* comme coefficient de corrélation **r=r[$\Delta$T,mABP],** à partir des valeurs $\Delta$T( mABP ) auxdits au moins deux cycles d'inflation-déflation ;

• **(g)** afficher le statut CA estimé.

9. Système selon la revendication 8, dans lequel le moyen de surveillance CA pour l'enregistrement continu des réponses transitoires CA est un moniteur CA ayant une résolution temporelle inférieure à la seconde :

   • un moniteur CA non invasif, tel que le Doppler transcrânien (3), ou le moniteur à temps de vol ultrasonique, ou le moniteur à spectroscopie proche infrarouge (NIRS) ;
   • ou/et est connecté pour enregistrer les données de surveillance d'un moniteur de pression intracrânienne invasive (PIC) (4).

10. Système selon la revendication 8, dans lequel la mABP est une moyenne du signal ABP(t) moyenné dans une fenêtre temporelle ayant une longueur dans la plage de 1 à 3 secondes, à la fin du cycle de gonflage à pression externe et avant le cycle de dégonflage à pression externe.

11. Système selon la revendication 8, dans lequel lesdits gonflages-dégonflages cycliques à pression externe *Pe*, appliqués pour modifier la pression artérielle du patient, présentent :

   • la durée du cycle de gonflage est de 1 à 3 minutes ;
   • la période du cycle de dégonflage est de 5 à 15 secondes.

12. Système selon la revendication 8, dans lequel lesdits gonflages-dégonflages cycliques à pression externe *Pe*, appliqués pour modifier la pression artérielle du patient, présentent :

   • des valeurs *Pe* contrôlées dans la plage de sécurité de 80 à 220 mmHg, pour une mABP lente, augmentant jusqu'à 5 à 10 mmHg au-dessus de la ligne de base de la mABP pendant le cycle de gonflage et une chute plus rapide de la mABP de 10 à 20 mmHg pendant le cycle de dégonflage.

13. Système selon la revendication 8, dans lequel, dans une seule mesure de l'état CA, au moins deux cycles de gonflage-dégonflage avec les différents niveaux de pression *Pe1, Pe2,* chacun est répété, de préférence, de 2 à 4 fois, et les valeurs AT et mABP estimées sont moyennées dans chaque groupe des cycles répétés.

14. Système selon la revendication 8, dans lequel le système indique le statut CA comme suit :

   **a)** si le délai de réponse transitoire $\Delta T$ est compris entre 2 et 5 secondes ou plus, le système indique une fonction CA intacte ; et
   **b)** si le délai de réponse transitoire $\Delta T$ est inférieur à 1,6 seconde, le système indique une fonction CA anormale, et en outre, sur la base des valeurs de signe *r* et *a* estimées, dans lequel

      • le signe *r* et *a* est négatif, le système indique de diminuer la valeur ABP ;
      • les signes *r* et *a* sont positifs, le système indique d'augmenter la valeur ABP.

15. Système selon la revendication 14, dans lequel le système indique en outre le statut de l'autorité de certification comme suit :

   • en cas de $\Delta T > 3$ secondes, aucune régulation de la mABP n'est requise ;
   • en cas de $\Delta T < 3$ secondes et de $\Delta T > 1,6$ seconde, pour que le statut CA et la régulation mABP atteignent la valeur $\Delta T$ maximale possible, ou la valeur mABP à laquelle $\Delta T$ est le maximum possible ; et
   • en cas de $\Delta T < 1,6$ seconde, pour le statut CA et la régulation continue de la mABP jusqu'à ce qu'une valeur $\Delta T = 1,6$ seconde au moins soit atteinte.

Figure 1

Figure 2

**Figure 3**

**Figure 4**

Figure 5

Figure 6

Mann-Whitney U-test p1=0.0032   p2<0.001  p3=0.015  /8

**Figure 7**

**Figure 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2023109678 A1

**Non-patent literature cited in the description**

- **PETKUS V** ; **PREIKSAITIS A** ; **CHALECKAS E** ; **CHOMSKIS R** ; **ZUBAVICIUTE E** ; **VOSYLIUS S** ; **ROCKA S** ; **RASTENYTE D** ; **ARIES MJ** ; **RAGAUSKAS A**. Optimal Cerebral Perfusion Pressure: Targeted Treatment for Severe Traumatic Brain Injury.. *J Neurotrauma.*, 13 November 2019, vol. 37 (2), 389-396
- **KUMPAITIENE B** ; **SVAGZDIENE M** ; **SIRVINSKAS E** ; **ADOMAITIENE V** ; **PETKUS V** ; **ZAKELIS R** ; **KRAKAUSKAITE S** ; **CHOMSKIS R** ; **RAGAUSKAS A** ; **BENETIS R**. Cerebrovascular autoregulation impairments during cardiac surgery with cardiopulmonary bypass are related to postoperative cognitive deterioration: prospective observational study.. *Minerva Anestesiol.*, 11 May 2018, vol. 85 (6), 594-603
- **DEIMANTAVICIUS M** ; **CHALECKAS E** ; **BOERE K** ; **PUTNYNAITE V** ; **TAMOSUITIS T** ; **TAMASAUSKAS A** ; **KAVALIAUSKAS M** ; **ROCKA S** ; **PREIKSAITIS A** ; **VOSYLIUS S**. Feasibility of the optimal cerebral perfusion pressure value identification without a delay that is too long.. *Sci Rep.*, 22 October 2022, vol. 12 (1), 17724

- **STEINER LA et al.** Continuous monitoring of cerebrovascular pressure reactivity allows determination of optimal cerebral perfusion pressure in patients with traumatic brain injury.. *Crit. Care Med.*, 2002, vol. 30, 733-738
- **OLSEN MH** ; **RIBERHOLT CG** ; **MEHLSEN J** ; **BERG RM** ; **MØLLER K**. Reliability and validity of the mean flow index (Mx) for assessing cerebral autoregulation in humans: A systematic review of the methodology.. *J Cereb Blood Flow Metab.*, 07 October 2021, vol. 42 (1), 27-38
- **OGOH, S.** ; **TARUMI, T.** Cerebral blood flow regulation and cognitive function: a role of arterial baroreflex function.. *J Physiol Sci*, 2019, vol. 69, 813-823, https://doi.org/10.1007/s12576-019-00704-6
- **SIMPSON DM** ; **PAYNE SJ** ; **PANERAI RB**. The INfoMATAS project: Methods for assessing cerebral autoregulation in stroke.. *Journal of Cerebral Blood Flow & Metabolism.*, 2022, vol. 42 (3), 411-429
- **AASLID R et al.** Cerebral autoregulation dynamics in humans.. *STROKE*, 01 January 1989, vol. 20 (1), 45-52